# EUROPÄISCHE PATENTSCHRIFT

(11) **EP 0 937 041 B1**
(45) Veröffentlichungstag und Bekanntmachung des Hinweises auf die Patenterteilung: **23.04.2003**
(21) Anmeldenummer: 97911049.1
(22) Anmeldetag: 11.11.1997
(51) Int. Cl.: C07D 207/12, A61K 31/40

(54) **Verwendung eines pharmazeutisch geeigneten Salzes von (3R,2'R)-3-[(Cyclopentyl-hydroxyphenylacetyl)oxy]-1,1-dimethyl-pyrrolidinium zur Herstellung eines Arzneimittels**
Use of a pharmaceutically acceptable salt of (3R,2'R)-3-[(Cyclopently-hydroxyphenylacetyl)oxy]-1,1-dimethyl-pyrrolidinium for the preparation of a medicament
Utilisation d'un sel pharmaceutiquement acceptable de (3R,2'R)-3-[(cyclopentyl-hydroxyphenylacetyl)oxy]1,1-dimethyl-pyrrolidimium pour la préparation d'un médicament

(30) Priorität: 11.11.1996 AT 197396
(43) Veröffentlichungstag der Anmeldung: 25.08.1999
(73) Patentinhaber: Noe, Christian R., 60596 Frankfurt am Main (DE); Mutschler, Ernst, 55129 Mainz (DE)
(72) Erfinder: NOE, Christian, R., D-60596 Frankfurt am Main (DE); MUTSCHLER, Ernst, D-55129 Mainz (DE); LAMBRECHT, Günter, D-55129 Main (DE); ELGERT, Michael, D-60437 Frankfurt am Main (DE); CZECHE, Sittah, D-99867 Gotha (DE); WAELBROECK, Magali, B-1070 Brüssel (BE)
(74) Vertreter: Puchberger, Rolf
(86) Internationale Anmeldenummer: AT9700245
(87) Internationale Veröffentlichungsnummer: WO98021183

(56) Entgegenhaltungen:
- US-A- 2 956 062
- I. DEMIAN; D. GRIPSHOVER : "High -Performance Liquid Chromatographic separation of 3-[(cyclopentylhydroxyphenylacetyl)oxy]-1, 1-dimethylpyrrolidinium bromide diastereoisomers" J. LIQ. CHROMATOGR., Bd. 13, Nr. 4, 1990, Seiten 779-87, XP002055746 in der Anmeldung erwähnt
- CHEMICAL ABSTRACTS, vol. 123, no. 5, 31.Juli 1995 Columbus, Ohio, US; abstract no. 47690y, Seite 58; XP002055748 & GOMEZ ET AL.: "Atropine and glycopyrronium show similar binding patterns to M2 and M3 muscarinic receptor subtypes in the rat" BR. J. ANAESTH., Bd. 74, Nr. 5, 1995, Seiten 549-52,
- CHEMICAL ABSTRACTS, vol. 119, no. 11, 13.September 1993 Columbus, Ohio, US; abstract no. 108934x, Seite 82; XP002055749 & FUDER ET AL.: "Glycopyrronium bromide blocks differentially responses mediated by muscarinic receptor subtypes" NAUNYN-SCHMIEDEBERG'S ARCH. PHARMACOL., Bd. 347, Nr. 6, 1993, Seiten 591-5,
- ZHI WANG ET AL.: "Use of cyclodextrins as chiral selector for the chiral separation of anticholinergic drugs such as anisodamine and glycopyrronium in capillary zone electrophoresis" J. HIGH RESOL. CHROMATOGR., Bd. 19, Nr. 12, Dezember 1996, Seiten 697-99, XP002055747

## Beschreibung

Gegenstand der vorliegenden Erfindung ist die Verwendung eines pharmazeutisch geeigneten Salzes von (3R,2'R)-3-[(Cyclopentylhydroxyphenylacetyl)oxy]-1,1-dimethyl-pyrrolidinium zur Herstellung eines Arzneimittels.

Das Gebiet der Erfindung bezieht sich auf enantiomerenreine Ester, somit die (3R,2'R)-, (3S,2'R)-, (3R,2'S)- und (3S,2'S)-konfigurierten Enantiomere der allgemeinen Formel I, 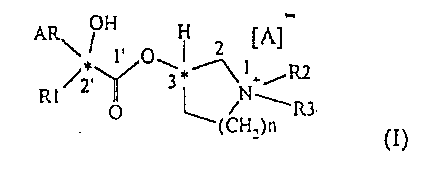 worin
- R₁: einen mono- bi oder tricyclischen C₃-C₉-Cycloalkylrest, der gegebenfalls durch einen oder mehrere C₁-C₆-Alkyl-, C₂-C₆-Alkenyl- und/oder C₂-C₆-Alkinylrest(e) und/oder durch ein oder mehrere Halogenatom(e) wie Fluor, Chlor, Brom oder Iod substituiert ist;
- R₂: einen C₁-C₆-Alkyl-, C₂-C₆-Alkenyl- oder C₂-C₆-Alkinylrest, der ggf. durch ein oder mehrere Halogenatom(e) wie Fluor, Chlor, Brom oder Iod substituiert sein kann;
- R₃: einen C₁-C₆-Alkyl-, C₂-C₆-Alkenyl- oder C₂-C₆-Alkinylrestdie ggf. durch ein oder mehrere Halogenatom(e) wie Fluor, Chlor, Brom oder Iod substituiert sein kann;
- AR: einen C₆-C₁₀-Aromaten oder einen Heteroaromaten, der Stickstoff, Schwefel oder Sauerstoff als Heteroatom enthält;
- n: eine ganze Zahl 1, 2 oder 3;
- A: ein Anion einer pharmakologisch unbedenklichen Säure
bedeuten können, Verfahren zu ihrer Herstellung und ihre Verwendung als Arzneimittel.

Bevorzugt sind Verbindungen der allgemeinen Formel I, in der
- R₁: einen mono- oder bicyclischen C₅-C₇-Cycloalkylrest, der gegebenenfalls durch einen oder mehrere C₁-C₃-Alkyl-, C₂-C₆-Alkenyl- oder C₂-C₆-Alkinylrest(e) und/oder durch ein oder mehrere Halogenatom(e) wie Fluor, Chlor, Brom oder Iod substituiert ist;
- R₂: einen C₁-C₃-Alkyl-, C₂-C₄-Alkenyl- oder C₂-C₄-Alkinylrest, der ggf. durch ein oder mehrere Halogenatom(e) wie Fluor, Chlor, Brom oder Iod substituiert sein kann;
- R₃: einen C₁-C₃-Alkyl-, C₂-C₄-Alkenyl- oder C₂-C₄-Alkinylrest, der ggf. durch ein oder mehrere Halogenatom(e) wie Fluor, Chlor, Brom oder lod substituiert sein kann;
- AR: einen C₆-C₁₀-Aromaten oder einen Heteroaromaten, der Schwefel als Heteroatom enthält;
- n: eine ganze Zahl 1 oder 2;
- A: ein Anion einer pharmakologisch unbedenklichen Mineralsäure oder einer Carbonsäure bedeuten können.

Besonders bevorzugt sind Verbindungen der allgemeinen Formel I, in der
- R₁: einen Cyclopentyl, einen Cyclohexyl oder einen Norbornylrest;
- R₂: einen Methylrest;
- R₃: einen Methylrest;
- AR: einen Phenylrest;
- n: eine ganze Zahl 1 oder 2;
- A: Fluorid, Chlorid, Bromid oder Jodid
bedeuten können und in welchen OH, AR und R₁ bei Blickrichtung gegen die Carboxylgruppe im Uhrzeigersinn angeordnet sind.

C₁-C₆-Alkyl steht - sofern nicht anders definiert - für einen verzweigten oder unverzweigten Alkylrest mit 1 bis 6 Kohlenstoffatomen, der gegebenenfalls mit einem oder mehreren Halogenatomen substituiert sein kann. - Im Sinne der der vorliegenden Erfindung sind beispielsweise folgende Substituenten als C₁-C₆-Alkylreste zu verstehen:
Methyl, Ethyl, Propyl, 1-Methylethyl (*iso*-Propyl), Butyl, 1-Methylpropyl, 2-Methylpropyl, 1,1-Dimethylethyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl.

Sofern nicht anders angegeben werden unter Alkylsubstituenten, die ein bis drei Kohlenstoffatome enthalten, vorzugsweise folgende Niederalkylreste verstanden:
Methyl, Ethyl, *n*-Propyl oder *iso*-Propyl.

C₂-C₆-Alkenyl steht - sofern nicht anders definiert - für einen verzweigten oder unverzweigten Alkylrest mit 2 bis 6 Kohlenstoffatmonen, der eine oder gegebenfalls zwei Doppelbindungen enthält gegebenenfalls mit einem oder mehreren Halogenatomen substituiert sein kann. - Im Sinne der vorliegenden Erfindung sind beispielsweise folgende Substituenten als C₂-C₆-Alkenylreste zu verstehen:
Vinyl, 2-Propenyl (Allyl), 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 3-Methyl-4-pentenyl, 4-Methyl-4-pentenyl, 1,1-Dimethyl-2-butenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, worunter der Allylrest bevorzugt ist.

C₂-C₆-Alkinyl steht - sofern nicht anders definiert - für einen verzweigten oder unverzweigten Alkylrest mit 2 bis 6 Kohlenstoffatomen, der eine oder gegebenfalls zwei Dreifachbindungen oder eine Dreifachbindung und eine Doppelbindung enthalten kann und gegebenenfalls mit einem oder mehreren Halogenatomen substituiert sein kann. - Im Sinne der vorliegenden Erfindung sind beispielsweise folgende Substituenten als C₂-C₆-Alkinylreste zu verstehen:
2-Propinyl (Propargyl), 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 4-Methyl-2-pentinyl, 1-Methyl-3-pentinyl, 2-Methyl-3-pentinyl, 1-Methyl-4-pentinyl, 3-Methyl-4-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl oder 1-Ethyl-1-methyl-2-propinyl, worunter der Propargylrest bevorzugt ist.

Cycloalkyl steht im allgemeinen für einen gesättigten oder ungesättigten cyclischen Kohlenwasserstoffrest mit 3 bis 9 Kohlenstoffatomen, der gegebenenfalls mit einem Halogenatom oder mehreren Halogenatomen - vorzugsweise Fluor - substituiert sein kann, die untereinander gleich oder verschieden sein können. Bevorzugt sind cyclische Kohlenwasserstoffe mit 5 oder 6 Kohlenstoffatomen. Als Beispiele seien Cyclopropyl, Cyclobutyl, Cyclopentyl, Cyclopentenyl, Cyclohexyl, Cyclohexenyl, Cycloheptyl, Cycloheptenyl, Cycloheptadienyl, Cyclooctyl, Cyclooctenyl, Cyclooctadienyl und Cyclononinyl genannt.

Aryl steht im allgemeinen für einen aromatischen Rest mit 6 bis 10 Kohlenstoffatomen - auch in Zusammensetzungen, wobei der Aromat mit einer oder mehreren Niederalkylgruppe(n) mit 1 bis 3 Kohlenstoffatomen, Trifluormethylgruppe(n), Cyanogruppe(n), Alkoxygruppe(n), Nitrogruppe(n), Aminogruppe(n) und/oder einem oder mehreren Halogenatom(en) - untereinander gleich oder verschieden - substituiert sein kann; bevorzugter Arylrest ist ein gegebenenfalls substituierter Phenylrest, wobei als Substituenten Halogen - wie Fluor, Chlor oder Brom - Cyano sowie Hydroxyl bevorzugt sind.

Heteroaryl im Rahmen der oben angegebenen Definition steht im allgemeinen für einen 5- bis 6-gliedrigen Ring, der als Heteroatome Sauerstoff, Schwefel und/oder Stickstoff enthalten kann und an den ein weiterer aromatischer Ring ankondensiert sein kann. Bevorzugt sind 5- und 6-gliedrige aromatische Ringe, die einen Sauerstoff, einen Schwefel und/oder bis zu zwei Stickstoffatomen enthalten und die gegebenenfalls benzokondensiert sind.

Als besondere heterocyclische Systeme seien beispielsweise Acridinyl, Acridonyl, Alkylpyridinyl, Anthrachinonyl, Ascorbyl, Azaazulenyl, Azabenzanthracenyl, Azabenzanthrenyl, Azachrysenyl, Azacyclazinyl, Azaindolyl, Azanaphthacenyl, Azanaphthalenyl, Azaprenyl, Azatriphenylenyl, Azepinyl, Azinoindolyl, Azinopyrrolyl, Benzacridinyl, Benzazapinyl, Benzofuryl, Benzonaphthyridinyl, Benzopyranonyl, Benzopyranyl, Benzopyronyl, Benzochinolinyl, Benzochinolizinyl, Benzothiepinyl, Benzothiophenyl, Benzylisoquinolinyl, Bipyridinyl, Butyrolactonyl, Caprolactamyl, Carbazolyl, Carbolinyl, Catechinyl, Chromenopyronyl, Chromonopyranyl, Cumarinyl, Cumaronyl, Decahydrochinolinyl, Decahydrochinolonyl, Diazaanthracenyl, Diazaphenanthrenyl, Dibenzazapinyl, Dibenzofuranyl, Dibenzothiphenyl, Dichromylenyl, Dihydrofuranyl, Dihydroisocumarinyl, Dihydroisochinolinyl, Dihydropyranyl, Dihydropyridinyl, Dihydropyridonyl, Dihydropyronyl, Dihydrothiopyranyl, Diprylenyl, Dioxanthylenyl, Oenantholactamyl, Flavanyl, Flavonyl, Fluoranyl, Fluoresceinyl, Furandionyl, Furanochromanyl, Furanonyl, Furanochinolinyl, Furanyl, Furopyranyl, Furopyronyl, Heteroazulenyl, Hexahydropyrazinoisoquinolinyl, Hydrofuranyl, Hydrofuranonyl, Hydroindolyl, Hydropyranyl, Hydropyridinyl, Hydropyrrolyl, Hydrochinolinyl, Hydrothiochromenyl, Hydrothiophenyl, Indolizidinyl, Indolizinyl, Indolonyl, Isatinyl, Isatogenyl, Isobenzofurandionyl, Isobenzfuranyl, Isochromanyl, Isoflavonyl, Isoindolinyl, Isoindolobenzazapinyl, Isoindolyl, Isochinolinyl, Isochinuclidinyl, Lactamyl, Lactonyl, Maleimidyl, Monoazabenzonaphthenyl, Naphthalenyl, Naphthimidazopyridindionyl, Naphthindolizinedionyl, Naphthodihydropyranyl, Naphthofuranyl, Naphthyridinyl, Oxepinyl, Oxindolyl, Oxolenyl, Perhydroazolopyridinyl, Perhydroindolyl, Phenanthrachinonyl, Phthalideisoquinolinyl, Phthalimidyl, Phthalonyl, Piperidinyl, Piperidonyl, Prolinyl, Pyrazinyl, Pyranoazinyl, Pyranoazolyl, Pyranopyrandionyl, Pyranopyridinyl, Pyranochinolinyl, Pyranopyrazinyl, Pyranyl, Pyrazolopyridinyl, Pyridinethionyl, Pyridinonaphthalenyl, Pyridinopyridinyl, Pyridinyl, Pyridocolinyl, Pyridoindolyl, Pyridopyridinyl, Pyridopyrimidinyl, Pyridopyrrolyl, Pyridochinolinyl, Pyronyl, Pyrrocolinyl, Pyrrolidinyl, Pyrrolizidinyl, Pyrrolizinyl, Pyrrolodioazinyl, Pyrrolonyl, Pyrrolopyrmidinyl, Pyrrolochinolonyl, Pyrrolyl, Chinacridonyl, Chinolinyl, Chinolizidinyl, Chinolizinyl, Chinolonyl, Chinuclidinyl, Rhodaminyl, Spirocumaranyl, Succinimidyl, Sulpholanyl, Sulpholenyl, Tetrahydrofuranyl, Tetrahydroisochinolinyl, Tetrahydropyranyl, Tetrahydropyridinyl, Tetrahydrothiapyranyl, Tetrahydrothiophenyl, Tetrahydrothipyranonyl, Tetrahydrothipyranyl, Tetronyl, Thiaphenyl, Thiachromanyl, Thiadecalinyl, Thianaphthenyl, Thiapyranyl, Thiapyronyl, Thiazolopyridinyl, Thienopyridinyl, Thienopyrrolyl, Thienothiophenyl, Thiepinyl, Thiochromenyl, Thiocumarinyl, Thiopyranyl, Triazaanthracenyl, Triazinoindolyl, Triazolopyridinyl, Tropanyl, Xanthenyl, Xanthonyl, Xanthydrolyl, Adeninyl, Alloxanyl, Alloxazinyl, Anthranilyl, Azabenzanthrenyl, Azabenzonaphthenyl, Azanaphthacenyl, Azaphenoxazinyl, Azapurinyl, Azinyl, Azoloazinyl, Azolyl, Barbituric Acid, Benzazinyl, Benzimidazolethionyl, Benzimidazolonyl, Benzisothiazolyl, Benzisoxazolyl, Benzocinnolinyl, Benzodiazocinyl, Benzodioxolanyl; Benzodioxolyl, Benzopyridazinyl, Benzothiazepinyl, Benzothiazinyl, Benzothiazolyl, Benzoxazinyl, Benzoxazolinonyl, Benzoxazolyl, Cinnolinyl, Depsidinyl, Diazaphenanthrenyl, Diazepinyl, Diazinyl, Dibenzoxazepinyl, Dihydrobenzimidazolyl, Dihydrobenzothiazinyl, Dihydrooxazolyl, Dihydropyridazinyl, Dihydropyrimidinyl, Dihydrothiazinyl, Dioxanyl, Dioxenyl, Dioxepinyl, Dioxinonyl, Dioxolanyl, Dioxolonyl, Dioxopiperazinyl, Dipyrimidopyrazinyl, Dithiolanyl, Dithiolenyl, Dithiolyl, Flavinyl, Furopyrimidinyl, Glycocyamidinyl, Guaninyl, Hexahydropyrazinoisoquinolinyl, Hexahydropyridazinyl, Hydantoinyl, Hydroimidazolyl, Hydroparazinyl, Hydropyrazolyl, Hydropyridazinyl, Hydropyrimidinyl, Imidazolinyl, Imidazolyl, Imidazoquinazolinyl, Imidazothiazolyl, Indazolebenzopyrazolyl, Indoxazenyl, Inosinyl, Isoalloxazinyl, Isothiazolyl, Isoxazolidinyl, Isoxazolinonyl, Isoxazolinyl, Isoxazolonyl. Isoxazolyl, Lumazinyl, Methylthyminyl, Methyluracilyl, Morpholinyl, Naphthimidazolyl, Oroticyl, Oxathianyl, Oxathiolanyl, Oxazinonyl, Oxazolidinonyl, Oxazolidinyl, Oxazolidonyl, Oxazolinonyl, Oxazolinyl, Oxazolonyl, Oxazolopyrimidinyl, Oxazolyl, Perhydrocinnolinyl, Perhydropyrroloazinyl, Perhydropyrrolothiazinyl, Perhydrothiazinonyl, Perimidinyl, Phenazinyl, Phenothiazinyl, Phenoxathiinyl, Phenoxazinyl, Phenoxazonyl, Phthalazinyl, Piperazindionyl, Piperazinodionyl, Polyquinoxalinyl, Pteridinyl, Pterinyl, Purinyl, Pyrazinyl, Pyrazolidinyl, Pyrazolidonyl, Pyrazolinonyl, Parazolinyl, Pyrazolobenzodiazepinyl, Pyrazolonyl, Pyrazolopyrimidinyl, Pyrazolotriazinyl, Pyrazolyl, Pyridazinyl, Pyridazonyl, Pyridopyrazinyl, Pyridopyrimidinyl, Pyrimidinethionyl, Pyrimidinyl, Pyrimidionyl, Pyrimidoazepinyl, Pyrimidopteridinyl, Pyrrolobenzodiazepinyl, Pyrrolodiazinyl, Pyrrolopyrimidinyl, Chinazolidinyl, Chinazolinonyl, Chinazolinyl, Chinoxalinyl, Sultamyl, Sultinyl, Sultonyl, Tetrahydrooxazolyl, Tetrahydropyrazinyl, Tetrahydropyridazinyl, Tetrahydroquinoxalinyl, Tetrahydrothiazolyl, Thiazepinyl, Thiazinyl, Thiazolidinonyl, Thiazolidinyl, Thiazolinonyl, Thiazolinyl, Thiazolobenzimidazolyl, Thiazolyl, Thienopyrimidinyl, Thiazolidinonyl, Thyminyl, Triazolopyrimidinyl, Uracilyl, Xanthinyl, Xylitolyl, Azabenzonapththenyl, Benzofuroxanyl, Benzothiadiazinyl, Benzotriazepinonyl, Benzotriazolyl, Benzoxadiazinyl, Dioxadiazinyl, Dithiadazolyl, Dithiazolyl, Furazanyl, Furoxanyl, Hydrotriazolyl, Hydroxytrizinyl, Oxadiazinyl, Oxadiazolyl, Oxathiazinonyl, Oxatriazolyl, Pentazinyl, Pentazolyl, Petrazinyl, Polyoxadiazolyl, Sydonyl, Tetraoxanyl, Tetrazepinyl, Tetrazinyl, Tetrazolyl, Thiadiazinyl, Thiadiazolinyl, Thiadiazolyl, Thiadioxazinyl, Thiatriazinyl, Thiatriazolyl, Thiatriazolyl, Triazepinyl, Triazinoindolyl, Triazinyl, Triazolinedionyl, Triazolinyl, Triazolyl, Trioxanyl, Triphenodioxazinyl, Triphenodithiazinyl, Trithiadiazepinyl, Trithianyl, oder Trioxolanyl genannt.

Als pharmazeutisch geeignetes Salz wird das Salz einer pharmakologisch unbedenklichen Säure bezeichnet.

Die erfindungsgemäß verwendeten enantiomerenreinen Verbindungen der allgemeinen Formel I können nach Verfahren hergestellt werden, die an sich aus dem Stand der Technik bekannt sind. Die essentiellen Schritte des Herstellungsverfahrens bestehen insbesondere darin, daß man eine enantiomerenreine α-Hydroxycarbonsäure (R- oder S- Enantiomer) der allgemeinen Formel II, 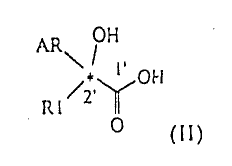 worin R₁ und AR die zuvor genannte Bedeutung haben, oder ihren Ester, bevorzugt einen C₁ - C₃ Alkylester, oder ein aktiviertes Säurederivat mit einem enantiomerenreinen Alkohol der allgemeinen Formel III (R- oder S-Enantiomer), 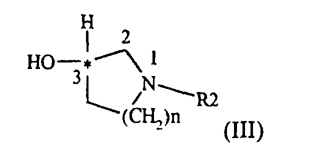 worin R₂ und n die vorgenannte Bedeutung haben, umsetzt und so erhaltenen enantiomerenreinen Ester der allgemeinen Formel IV 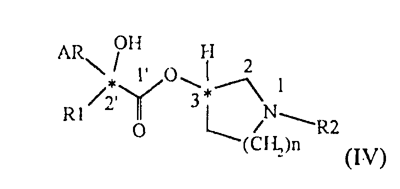 mit einem Alkylierungsmittel der allgemeinen Formel V,

R₃ - X (V)

in der X eine gegen eine tertiäre Aminogruppe substituierbare Austrittsgruppe verkörpert, umsetzt und das resultierende Salz isoliert bzw. umsalzt.

Im bevorzugten Verfahren wird die Säure der allgemeinen Formel II in razemischer Form mit der enantiomerenreinen Aminoalkoholkomponente der allgemeinen Formel III umgesetzt und das resultierende Diastereomerengemisch nach an sich aus dem Stand der Technik bekannten Verfahren - insbesondere auf dem Wege der Kristallisation - bevorzugt unter Verwendung einer enantiomerenreinen Hilfssäure - getrennt.

Ester von Aryl-cycloalkyl-hydroxysäuren mit cyclischen Alkoholen, in welchen ein quartärer Stickstoff vorhanden ist, und welche mit der allgemeinen Formel I 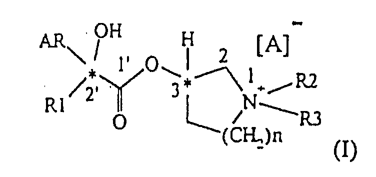 beschrieben werden, bestehend aus einer Hydroxycarbonsäure, in welcher AR einen aromatischen Ring bedeutet und in welcher R₁ einen cycloaliphatischen Ring bedeutet und bestehend aus einer Alkoholkomponente, in welcher sich die Hydroxylgruppe an einem Dimethylpyrrolidiniumring (n=1) oder Dimethylpiperidiniumring (n=2) befindet, in welchem R₂ = R₃ ein Niederalkyl bedeutet, und in welcher A ein Halogenid bedeutet, wurden in einigen Fällen als Spasmolytika beschrieben. Wenn die beiden Reste R₂ und R₃ identisch sind, weisen Verbindungen der allgemeinen Formel I zwei Chiralitätszentren auf. Das eine Zentrum ist dem Säureteil zuzuordnen und betrifft die mit 2' bezeichnete Position, das zweite Chiralitätszentrum befindet sich im cyclischen Ringsystem an der mit 3 bezeichneten Position. Da Verbindungen dieser Struktur somit zwei Chiralitätszentren aufweisen, sind im Prinzip vier Stereoisomere (3R,2'R; 3S,2'R; 3R,2'S und 3S,2'S) denkbar. Bisher wurden reine Stereoisomere der allgemeinen Formel I weder isoliert noch synthetisch hergestellt, oder - was für den Gegenstand der vorliegenden Erfindung grundlegend ist - pharmakologisch untersucht. Der wichtigste, auch in der Therapie eingesetzte Vertreter der allgemeinen Formel I ist Glycopyrroniumbromid (AR = Phenyl, R₁ = Cyclopentyl, R₂ = R₃ = Methyl, n = 1, A = Br). Mit dem internationalen Freinamen Glycopyrroniumbromid wird das razemische Diastereoisomerengemisch, in welchem also alle vier Stereoisomere enthalten sind, beschrieben.

Bisher bekanntgewordene Publikationen und Patente befassen sich entweder mit dem als Stereoisomerengemisch vorliegenden Wirkstoff Glycopyrroniumbromid (CAS 596-51-0), mit erythro- (RN 59677-73-5) bzw. threo- (RN 59677-70-2) konfigurierten Racematen des tert. Aminoesters (CRN 131118-11-1), welche lediglich als Vorstufe bei der Synthese der Verbindungen der Formel I angesehen werden können, oder dem Stereoisomerengemisch des analogen Cyclohexyl-Derivates (R₁ = Cyclohexyl) der allgemeinen Formel I (mit n = 1) (RN 101564-29-8). In den Publikationen zu den Chemical Abstracts Zitaten 80:53209h und 80:53214f sind Ergebnisse von Kristallstrukturanalysen der als Stereoisomerengemische vorliegenden Verbindungen Glycopyrroniumbromid und Hexapyrroniumbromid beschrieben. Die Publikationen zu den Chemical Abstracts Zitaten 80: 66587e, 80:66588f und 89:191258 beschreiben Ergebnisse von pharmakologischen Untersuchungen mit dem Stereoisomerengemisch der Verbindung Glycopyrroniumbromid bzw. von Kombinationspräparaten dieser Substanz mit Neostigmin und Pyridostigmin. Die Publikationen zu den Abstracts 84:43164h und 85:32832u beschreiben die teilweise Trennung des Sterosiomerengemisches auf dem Wege einer Kristallisation mit 5-Nitroisophthalsäure und die NMR-Untersuchung der bereits oben erwähnten threo- bzw. erythrokonfigurierten Racemate. Hierbei gelang den Autoren, ausgehend vom Stereoisomerengemisch (CRN 131118-11-1), lediglich die Diastereomerentrennung in die zwei Razemate, jedoch keine Herstellung der enantiomerenreinen Verbindungen. Die Publikationen zu den Chemical Abstracts Zitaten 96:29498m, 105:48928x, 113:158782t, 89: 191258k, sowie das europäische Patent EP 128886 A2 beschreiben Ergebnisse von Studien zur chromatographischen Analytik des Stereoisomerengemisches der Verbindung Glycopyrroniumbromid bzw. die Herstellung der verwendeten stationären Phasen. In keiner der aufgeführten Publikationen wird eine Enantiomerentrennung bzw. Isolierung der einzelnen Stereoisomere der allgemeinen Formel I berichtet. Eine HPL-chromatographische Trennung gelang in allen aufgeführten Fällen nur auf der Stufe der Diastereomeren. Die Herstellung der in der Patentanmeldung beanspruchten enantiomerenreinen Verbindungen der allgemeinen Formel I ist aus dem Stand der Technik noch nicht bekannt.

Die pharmakologische Wirkung von Arzneistoffen der allgemeinen Formel I basiert auf ihrer Interaktion mit muskarinischen Acetylcholinrezeptoren (Muskarinrezeptoren). Sie werden daher als m-Cholinozeptor-Antagonisten, bzw. Parasympatholytika oder - wegen ihrer erschlaffenden Wirkung auf die glatte Muskulatur - als neurotrope Spasmolytika bezeichnet. Die vielfältigen Wirkungen der Parasympatholytika umfassen: Beschleunigung der Herzfrequenz, Reduktion der Sekretion der Tränen-, Speichel- und Schweiß-Sekretion sowie der Drüsen des Verdauungstraktes, Erschlaffung der glatten Muskulatur der Bronchien, des Magen-Darm-Kanals, der Gallenwege, Uteren und der Harnblase, Erweiterung der Pupillen und Akkommodationsstörung. Quartäre Spasmolytika, zu welchen auch die Verbindungen gemäß der allgemeinen Formel I gehören, überwinden die Blut-Hirn-Schranke nicht und sind daher zentral unwirksam. Je nach Art der Anwendung sind die gewünschten und unerwünschten Wirkungen von Parasympatholytika verschieden. Verwendet man diese Substanzen als Spasmolytika, so wird man beispielsweise die verminderte Speichelsekretion oder die Pupillenerweiterung als Nebenwirkung bezeichnen.

Aufgrund der Forschungen der letzten Jahre ist es bekannt, daß Muskarinrezeptoren keine einheitliche Struktur besitzen, sondern daß die pharmakologischen Wirkungen auf Interaktionen mit mindestens vier verschiedenen Muskarinrezeptor-Subtypen zurückzuführen sind. Diese weisen einerseits eine unterschiedliche Verteilung in verschiedenen Organen auf, andererseits sind bei manchen neuronalen Signalübertragungskaskaden verschiedene Muskarinrezeptor-Subtypen mit verschiedenen Funktionen involviert. Verschiedene Wirkungen bzw. Nebenwirkungen lassen sich auf Interaktionen mit den verschiedenen Rezeptorsubtypen zurückführen, so daß eine hohe Subtypspezifität ein Ziel bei der Entwicklung moderner Spasmolytika ist. Glycopyrroniumbromid ist ein lange etablierter Wirkstoff, der den Anforderungen eines "modernen" Therapeutikums dieses Typs nicht entspricht. Glycopyrroniumbromid ist jedoch nicht nur ein Razemat, sondern darüber hinaus ein Diastereomerengemisch, bei welchem je nach Herstellungsprozedur die Verhältnisse der einzelnen Isomere im Produkt sogar schwanken können. Somit kann es bei solchen isomeren Wirkstoffgemischen zu zufälligen Subtypprofilen kommen, wodurch ein gezielter Einsatz erschwert und das Auftreten von unerwünschten Nebenwirkungen provoziert wird.

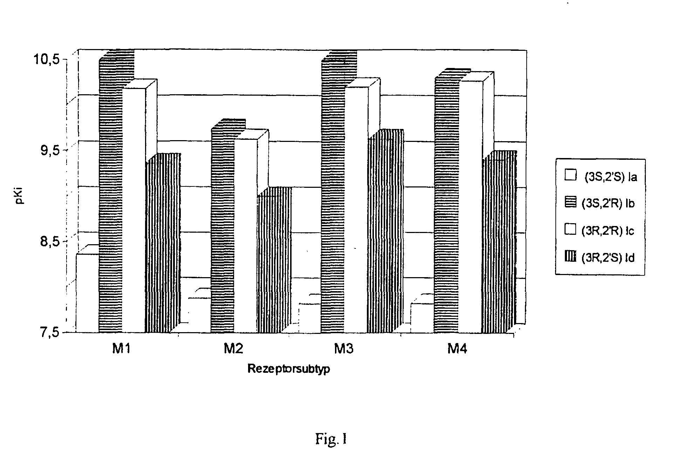

Häufig liegt bei der Trennung des Razemates eines Arzneistoffes in Enantiomere die pharmakologische Wirkung ausschließlich bei einem der Enantiomere. Aus Beispiel 9, vor allem aus der logarithmisch aufgetragenen Graphik Fig 1, kann entnommen werden, daß bei Verbindungen gemäß der vorliegenden Patentanmeldung alle Isomere im Prinzip Rezeptoraffinität aufweisen können. Jedoch zeigen die einzelnen Enantiomere zum einen deutliche Unterschiede in ihren Affinitäten, zum anderen ergeben sich auch deutliche Abweichungen in der Subtypspezifität M₁ - M₄, wobei die Unterschiede in den Affinitäten maximal einen Faktor von etwa 1000 ausmachen. Gerade die hohe Affinität zum M₃-Rezeptor-Subtyp bei einer relativ niederen Affinität zum M₂-Rezeptor-Subtyp macht die bevorzugt beanspruchten höher affinen Enantiomere (etwa in Beispiel 9: 1b und 1c) zu besonders geeigneten Wirkstoffen zur Therapie von Spasmen der glatten Muskulatur des Magen-Darm-Traktes und des Urogenitaltraktes sowie zur Behandlung obstruktiver Atemwegserkrankungen. Durch ein besonders günstiges Subtypprofil und eine durch ihre hohe Affinität bedingte besonders niedrige Dosierungsmöglichkeit bringen sie einen effizienteren therapeutischen Erfolg bei deutlich reduziertem Nebenwirkungspotential.

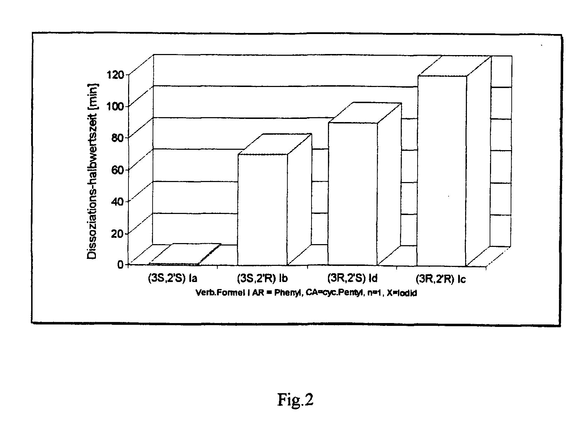

Ein weiterer besonders wichtiger Faktor bei der therapeutischen Anwendung von Enantiomeren der allgemeinen Formel I besteht in der kinetischen Subtypselektivität. Wie Beispiel 10, bzw. Fig. 2 entnommen werden kann, liegen die Dissoziationshalbwertszeiten der einzelnen Enantiomere 1a - 1d der Verbindung der allgemeinen Formel I (AR = Phenyl, R₁ = Cyclopentyl, R₂ = R₃ = Methyl, n = 1, A = 1) beim M₃-Rezeptorsubtyp zwischen 1 Minute und 120 Minuten, während die Dissoziationshalbwertzeiten an den Subtypen M₁, M₂ und M₄ im Bereich von wenigen Minuten liegen. Gerade Verbindungen mit einer besonders langen Dissoziationshalbwertszeit erlauben wegen ihrer starken Haftung eine besonders niedere Dosierung bei lange anhaltendem therapeutischem Effekt. Die Möglichkeit, durch gezielte Auswahl eines Enantiomers mit bestimmter Dissoziationshalbwertszeit die Dauer der pharmakologischen Wirkung gezielt zu beeinflussen, stellt einen weiteren wichtigen Fortschritt der Verbindungen der vorliegenden Patentanmeldung gegenüber dem Stand der Technik dar. Die beschriebenen Eigenschaften waren nicht vorhersehbar, auch gab es keine Hinweise in der Literatur.

Zusammenfassend läßt sich feststellen, daß sich enantiomerenreine Ester der allgemeinen Formel I gegenüber dem Stand der Technik durch ihre pharmakodynamische Selektivität auszeichnen. Sie besitzen in der bevorzugt beanspruchten Konfiguration eine deutlich höhere Affinität zu muskarinischen M₃- als zu M₂-Rezeptoren und zeigen darüber hinaus eine kinetische Selektivität für M₃-Rezeptoren, d.h. sie diffundieren nur langsam von diesem Rezeptortyp. Aufgrund dieser Eigenschaften eignen sie sich ganz besonders zur Therapie von Spasmen der glatten Muskulatur des Magen-Darm-Kanals und des Urogenitaltraktes sowie zur Behandlung obstruktiver Atemwegserkrankungen, wie Asthma bronchiale und chronische Bronchitis. Im Vergleich zu den bisher angewandten, nichtselektiven Parasympatholytika weisen sie aufgrund ihrer definierten Subtypselektivität deutliche Unterschiede in den pharmakologischen Eigenschaften auf. Im Vergleich zu bekannten Stereoisomerengemischen oder Razematen können die Verbindungen zudem in besonders niedriger Dosierung eingesetzt werden (Vermeidung von enantiomerem Ballast!). Aus diesen Gründen sind Nebenwirkungen mit Sicherheit in deutlich geringerem Umfang zu erwarten.

Demgemäß ist die Verwendung von enantiomerenreinen Estern (3R,2'R-, 3S,2'R-, 3R,2'S- bzw. 3S,2'S-Enantiomer) der allgemeinen Formel I in Arzneimitteln zur Therapie von Spasmen der glatten Muskulatur des Magen-Darm-Kanals und des Urogenitaltraktes sowie zur Behandlung obstruktiver Atemwegserkrankungen (Asthma bronchiale, chronische Bronchitis) ein bevorzugter Gegenstand der Erfindung.

Besonders bevorzugt ist die Verwendung von Enantiomeren der allgemeinen Formel I mit hoher M₃-Subtypselektivität (pKᵢ größer als 10) und großen Dissoziationshalbwertszeiten am M₃-Rezeptor in Arzneimitteln zur Behandlung obstruktiver Atemwegserkrankungen, bevorzugt Asthma bronchiale und chronische Bronchitis.

Die beschriebene Erfindung wird nunmehr durch die folgenden Beispiele erläutert. Verschiedenartige, andere Ausgestaltungen werden für den Fachmann aus der vorliegenden Beschreibung ersichtlich. Es wird jedoch ausdrücklich darauf hingewiesen, daß die Beispiele und die Beschreibung lediglich zur Erläuterung vorgesehen und nicht als Einschränkung der Erfindung anzusehen sind.

### Beispiele:

### Beispiel 1:

### Herstellung von (3S,2'S) 3-[(cyclopentylhydroxyphenylacetyl)oxy]-1,1-dimethyl-pyrrolidinium-iodid 1a (allg.Formel I, Ar = Phenyl, R₁ = Cyclopentyl, R₂ = R₃ = Methyl, n=1, A = I).

In einer trockenen Reaktionsapparatur werden 20 mmol) (3S)-1-Methyl-3-pyrrolidinol und 24 mmol 2-Cyclopentyl-2-hydroxy-phenylessigsäuremethylester in 500 ml n-Heptan abs. vorgelegt. Anschließend werden 200 ml Heptan zum Entfernen aller Feuchtigkeitsspuren überdestilliert und durch den Wasserabscheider abgelassen. Nach dem Abkühlen werden 2 mmol NaH oder NaOMe (10 mol%) zugesetzt und wiederum zum Sieden erhitzt. Die Temperatur wird so gewählt, daß das n-Heptan nur langsam überdestilliert. Die übergehende Menge wird über 5-6 h fortlaufend aus dem Tropftrichter ersetzt, bis der Hydroxyester vollständig umgesetzt ist. Nach wäßriger Aufarbeitung des Reaktionsgemisches und Extraktion mit Ether wird das Rohprodukt über Na₂SO₄ / K₂CO₃ 2: 1 getrocknet. Nach Absaugen vom Trockenmittel wird im Eisbad vorgekühlt und unter Eiskühlung bis zur Sättigung mit etherischer HCl / 2-Butanon versetzt. Hierbei fällt das Produkt zunächst ölig an. Durch Zusatz von 2-Butanon bzw. durch Abdestillieren von Ether erhält man eine klare Lösung, aus der unter Eiskühlung das Hydrochlorid des Diasteromerengemisches (3S,2'R/S)- 3-[(cyclopentylhydroxyphenylacetyl)oxy]-1-methyl-pyrrolidin (allg. Formel IV Ar = Phenyl, R₁ = Cyclopentyl, R₂ = Methyl, n = 1) auskristallisiert. Ausbeute 15,7 mmol. Fp. 176°C.

### Herstellung der Hydrogentartrate: Diastereomerentrennung durch fraktionierende Kristallisation:

Zur Herstellung der Hydrogentartrate überführt man 15 mmol des oben beschriebenen Hydrochlorids mit-NaHCO₃ / K₂CO₃ Puffer pH 10 in einen Scheidetrichter und extrahiert die wäßrige Phase dreimal mit je 150 ml Diethylether. Die vereinten etherischen Phasen werden mit 100 ml Ethylacetat versetzt und über Na₂SO₄ / K₂CO₃ 2:1 getrocknet. Nach Absaugen vom Trockenmittel wird am Rotationsverdampfer auf ca. 100 ml Volumen reduziert. Die Lösung wird auf ca. 60°C erhitzt und mit einer Lösung von 1,2 eq (18mmol) enantiomerenreiner Weinsäure in Ethylacetat versetzt. Das Hydrogentartrat kristallisiert über Nacht im Kühlschrank. Durch mehrfaches Umkristallisieren lassen sich die Diastereomeren bis zu einem de von 99% trennen. Ausbeute 8,3 mmol
(3S,2'S) 3-[(cyclopentylhydroxyphenylacetyl)oxy]-1-methyl-pyrrolidin, D-(-)-Hydrogentartrat allg. Formel IV (AR = Phenyl, R₁ = Cyclopentyl, R₂ = Methyl, n = 1): Smp.178-179°C ¹H-NMR-Spektrum (300 MHz CD₄O): δ (ppm) 1,3-1,7 (M,8H,Cyclopentyl-CH₂), 2,05-2,1 (M,1H,C4), 2,39-2,46 (M,1H,C4), 2,77 (S,3H,N-Methyl), 2,97-3,0 (M,1H,Cyclopentyl C1), 3,18-3,25 (dd,1H,C2, ²J =12,8 Hz,³J=0-1 Hz), 3,31-3,5 (M,2H,C5), 3,6-3,7 (dd,1H,3J=5,2Hz,2J=13Hz,C2), 4,42 (S,2H,Tartrat), 5,34-5,39 (M,1H,C3), 7,2-7,8 (M,5H) Zuordnung aufgrund H,H-COSY-NMR

### Quarternierung:

Nach Freisetzen der Basen durch Extraktion mit Ether gegen Bicarbonat-Puffer pH 10 und Trocknen über Na₂SO₄ / K₂CO₃ 2:1 wird durch Zugabe von 3eq (20 mmol) Methyliodid quarterniert und das kristallin anfallende Produkt (3S,2'S)-3-[(cyclopentylhydroxyphenylacetyl)oxy]-1,1-dimethylpyrrolidinium-iodid 1a (allg.Formel I, AR = Phenyl, R₁ = Cyclopentyl, R₂ = R₃ = Methyl, n = 1, A = I) abgesaugt. (Die Bestimmung der Diastereomerenüberschüsse kann durch HPLC-Methoden an β-Cyclodextrin- und "Whelck"-Phasen bzw. durch Auswertung von NMR-Spektren der oben beschriebenen Hydrogentartrate erfolgen.)

(3 S,2'S)- 3-[(cyclopentylhydroxyphenylacetyl)oxy]-1,1-dimethyl-pyrrolidinium-iodid (1a) allg.Formel I (AR = Phenyl, R₁ = Cyclopentyl, R₂ = R₃ = Methyl, n = 1, A = I) Smp.165°C
¹H-NMR (300MHz CD₄O) δ (ppm) (1,15-1,4 (M,2H), 1,5-1,7 (M,6H),Cyclopentyl-Methylen), 2,2 (M,1H,C 4), 2,63 (M,1H,C 4), 2,9 (S,3H,N-Methyl), 2,93-2,99 (M,1H,Cyclopentyl-Methin), 3,1 (S,3H,N-Methyl), 3,43-3,47 (dd,1H,C2,²J=14Hz,³J=0Hz), 3,5-3,7 (M,2H,C5), 3,75 (dd,1H,C2,²J=13,7Hz,³J=6,05Hz), 5,38 (M,1H,C3), 7,15-7,4 (M,3H), 7,5-7,65 (M,2H)
¹³C-NMR 52 MHz CD₄O δ (ppm) (24,4-25,4) (4t,Cyclopentyl-methylen), 28,5 (t,C4), 47,4 (t,Cyclopentyl-Methin), 51,3 (q,N-Methyl), 51,8 (q,N-Methyl), 63,6 (t,C5), 68,9 (t,C2), 72,0 (d,C3), 78,4 (s,Hydroxyester C2), 124,5 (d), 126,1 (d), 126,7 (d), 140,5 (s), 172,4 (s)

Die Bestimmung der Diastereomerenreinheit (de) erfolgte durch Vergleich der Integrale der N-Methyl-protonen der diastereomeren Hydrogentartrate in ¹H-NMR-Spektren (300 MHz, CD₄O), bzw. durch HPLC-Analytik an β-Cyclodextrinphasen. (Cyclobond β-CD-OH, 50*0,4 cm, Puffer: 85%H₂O, 15% CH3CN, 0,2% CH3COOH V/V, 0,35 ml/min isokratisch, UV-Detektion: 236 nm).

### Beispiel 2:

### Herstellung von (3S,2'R) 3-[(cyclopentylhydroxyphenylacetyl)oxy]-1,1-dimethyl-pyrrolidinium-iodid 1b (allg.Formel I, Ar = Phenyl, R₁ = Cyclopentyl, R₂ = R₃ = Methyl, n = 1, A = I)

Aus den Mutterlaugen der unter Beispiel 1 beschriebenen Diastereomerentrennung wird durch Zusatz von Ether das D-(-)-Hydrogentartrat der (3S,2'R)-konfigurierten Verbindung kristallisiert. Mehrfaches Umkristallisieren führt zu einem de von > 98%.

(3S,2'R)-3-[(cyclopentylhydroxyphenylacetyl)oxy]-1-methyl-pyrrolidin, D-(-)-Hydrogentartrat (allg.Formel IV AR = Phenyl, R₁ = Cyclopentyl, R₂ = Methyl, n = 1): Smp.158-160°C, ¹H-NMR (300 MHz CD₄O): δ (ppm) 1,3-1,7 (M,8H,Cyclopentyl-CH₂), 2,0-2,1 (M,1H,C4), 2,39-2,46 (M,1H,C4), 2,81 (S,3H,N-Methyl), 2,93-3,05 (M,1H,Cyclopentyl C1), 3,24-3,4 (M,3H,C2,C5), 3,63-3,7 (dd,1H, ³J=5,2Hz, ²J=13Hz, C2), 4,42 (S,2H,Tartrat),5,38 (M,1H,C3), 7,2-7,7 (M,5H) Zuordnung aufgrund H,H-COSY-NMR.

Die Quarternierung erfolgte ebenfalls wie oben beschrieben und liefert das kristallin anfallende Produkt (3S,2'R) 3-[(cyclopentylhydroxyphenylacetyl)oxy]-1,1-dimethyl-pyrrolidinium-iodid 1b (allg.Formel I, AR = Phenyl, R₁ = Cyclopentyl, R₂ = R₃ = Methyl, n = 1, A = I). Smp. 108-109°C ¹H-NMR (300MHz CD₄O): δ (ppm) (1,15-1,4 (M,2H), 1,5-1,7 (M,6H),Cyclopentyl-Methylen), 2,2 (M,1H,C 4), 2,65-2,85 (M,1H,C 4), 3,01 (M,1H,Cyclopentyl-Methin), 3,06 (S,3H,N-Methyl), 3,1 (S,3H,N-Methyl), 3,55-3,8 (M,3H,C2,C5), 4,07 (dd,1H,C2,²J=13,8Hz,³J=6,2Hz), 5,48 (M,1H,C3), 7,26-7,4 (M,3H), 7,5-7,65 (M,2H).
¹³C-NMR (50 MHz CD₄O) / DEPT und CH-Korelation): δ (ppm) (27,0 (t), 27,4 (t), 27,41 (t), 28,06 (t),Cyclopentyl-methylen), 31,26 (t,C4), 46,6 (t,Cyclopentyl-Methin), 53,8 (q,N-Methyl), 54,3 (q,N-Methyl), 66,2 (t,C5), 71,5 (t,C2), 74,5 (d,C3), 81,2 (s,Hydroxyester C2), 127 (d), 128,8 (d), 129,3 5 (d), 143,2 (s), 175,0 (s).
Die Bestimmung der Diastereomerenreinheit erfolgte wie in Beispiel 1 beschrieben.

### Beispiel 3 (erfindungsgemäß):

### Herstellung von (3R,2'R)-3-[(cyclopentylhydroxyphenylacelyl)oxy]-1,1-dimethyl-pyrrolidiniumiodid 1c (allg.Formel I, Ar = Phenyl, R₁ = Cyclopentyl, R₂ = R₃ = Methyl, n = 1, A = I)

Die Herstellung erfolgte wie unter Beispiel 1 beschrieben, ausgehend von (3R)-1-Methyl-3-pyrrolidinol unter Verwendung von L(+)-Weinsäure zur Diastereomerentrennung. Das unter Beispiel 1 beschriebene Verfahren liefert (3R,2'R)- 3-[(cyclopentylhydroxyphenylacetyl)oxy]-1,1-dimethylpyrrolidinium-iodid 1c (allg. Formel I, Ar = Phenyl, R₁ = Cyclopentyl, R₂ = R₃ = Methyl, n = 1, A = I). Die [¹H]- und [¹³C]-NMR analytischen Daten stimmen mit jenen der in Beispiel 1 aufgeführten (3S,2'S)-konfigurierten Verbindung 1a überein. Smp.165-166°C
Die Bestimmung der Diastereomerenreinheit erfolgte wie in Beispiel 1 beschrieben.

### Beispiel 4:

### Herstellung von (3R,2'S) 3-[(cyclopentylhydroxyphenylacetyl)oxy]-1,1-dimethyl-pyrrolidinium-iodid 1d (allg.Formel I, Ar = Phenyl, R₁ = Cyclopentyl, R₂ = R₃ = Methyl, n = 1, A = I)

Aus den Mutterlaugen der unter Beispiel 1 beschriebenen Diastercomerentrennung der 3R-konfigurierten Verbindungen wird durch Zusatz von Ether das L-(+)-Hydrogentartrat der (3R,2'S)-konfigurierten Verbindung kristallisiert. Mehrfaches Umkristallisieren führt zu einem de von > 98%. Die Quarternierung erfolgt ebenfalls wie in Beispiel 1 beschrieben und liefert das kristallin anfallende Produkt (3R,2'S)- 3-[(cyclopentylhydroxyphenylacetyl)oxy]-1,1-dimethyl-pyrrolidinium-iodid 1d (allg.Formel I, AR = Phenyl, R₁ = Cyclopentyl, R₂ = R₃ = Methyl, n = 1, A = I). Die [¹H]- und [¹³C]-NMR analytischen Daten stimmen mit jenen der in Beispiel 2 aufgeführten (3S,2'R)-konfigurierten Verbindung 1b überein. Smp.107-108°C
Die Bestimmung der Diastereomerenreinheit erfolgte wie in Beispiel 1 beschrieben.

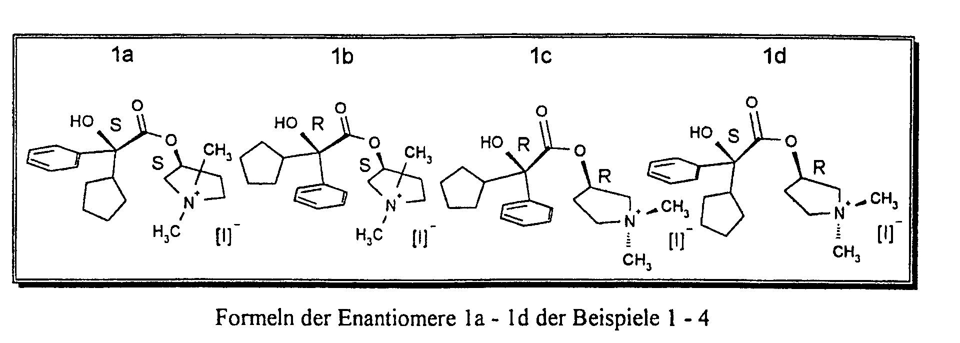

### Beispiel 5:

### Herstellung von (3S,2'S) 3-[(cyclohexylhydroxyphenylacetyl)oxy]-1,1-dimethyl-pyrrolidiniumbromid 2a (allg Formel I, Ar = Phenyl, R₁ = Cyclohexyl, R₂ = R₃ = Methyl, n=1, A = Br)

Die Herstellung der unter Beispiel 5 aufgeführten Verbindung erfolgt ausgehend von (3S)-1-Methyl-3-pyrrolidinol, 2-Cyclohexyl-2-hydroxy-phenylessigsäuremethylester und NaOMe nach dem unter Beispiel 1 beschriebenen Verfahren. Umesterung und Diastereomerentrennung der 3S-konfigurierten L (+)-Hydrogentartrate erfolgen in analoger Art und Weise.

(3S,2'S)- 3-[(cyclohexylhydroxyphenylacetyl)oxy]-1-methyl-pyrrolidin, L(+)-hydro-gentartrat: allg. Formel IV (AR = Phenyl, R₁ = Cyclopentyl, R₂ = Methyl n = 1): ¹H-NMR (300 MHz CD4O): δ (ppm) 1,12-1,2 (M,4H,Cyclohexyl-CH₂), 1,2-1,64 (M,3H,Cyclohexyl-CH₂), 1,64-1,67 (M,2H,Cyclohexyl-CH₂), 1,75-1,85 (M,1H,Cyclohexyl-CH₂), 2,03-2,1 (M,1H,C4), 2,25-2,4 (M,1H,Cyclohexyl-Methin), 2,40-2,55 (M,1H,C4), 2,78 (S,3H,N-Methyl), 3,22 (dd,1H,C2, ²J =13,3 Hz,³J=0-1 Hz), 3,27-3,51 (M,2H,C5), 3,65 (dd,1H,3J=5,42Hz, 2J=13, 2Hz,C2), 4,42 (S,2H,Tartrat), 5,37 (M,1H,C3), 7,2-7,61 (M,5H). Die Zuordnung erfolgte aufgrund H,H-COSY-NMR-Spektren. ¹³C-NMR (50 MHz CD₄O / DEPT): δ (ppm) (26,71 (t), 27,35(t), 27,47 (t), 28,71 (t),Cyclohexyl-Methylen), 31,8 (t,C4), 42,5 (q,N-Methyl), 46,92 (d,Cyclohexyl-methin), 55,35 (t,C5), 61,24 (t,C2), 74,2 (d,Tartrat-Methin), 75,2 (d,C3), 82,7 (s,Hydroxyester C2'), 126,9 (d), 128,7 (d), 129,2 (d), 142,1 (s), 175,0 (s,Tartrat-Carboxyl), 177,2 (s,Hydroxyester-Carboxyl).

Zur Quarternierung wird in Abwandlung der unter Beispiel 1 aufgeführten Vorschrift Methylbromid in tert.Butyl-methylether verwendet.

(3S, 2'S) 3-[(cyclohexylhydroxyphenylacetyl)oxy]-1,1-dimethyl-pyrrolidiniumbromid 2a: ¹H-NMR (300 MHz CD4O): δ (ppm) (1,1-2,0 (M,10H), Cyclohexyl-Methylen), 2,1-2,3 (M, 1H,cHex-Methin), 2,35-2,45 (M,1H,C4), 2,65-2,85 (M,1H,C4), 3,07 (S,3H,N-Methyl), 3,21 (S,3H,N-Methyl), 3,55-3,8 (M,3H,C2,C5), 3,85 (dd,1H,C2,²J=13,8Hz,³J=6,1Hz), 5,47 (M,1H,C3), 7,25-7,4 (M,3H), 7,55-7,65 (M,2H)
Die Bestimmung der Diastereomerenreinheit (de) erfolgte durch Vergleich der Integrale der N-Methyl-protonen der diastereomeren Hydrogentartrate.bei 2,78 ppm und 2,82 ppm in ¹H-NMR-Spektren (300 MHz, CD₄O).

### Beispiel 6:

### Herstellung von (3S,2'R) 3-[(cyclohexylhydroxyphenylacetyl)oxy]-1,1-dimethyl-pyrrolidiniumbromid 2b (allg.Formel I, Ar = Phenyl, R₁ = Cyclohexyl, R₂ = R₃ = Methyl, n = 1, A = Br)

Aus den Mutterlaugen der Diastereomerentrennung zur Herstellung von (3S,2'S) 3-[(cyclohexylhydroxyphenylacetyl)oxy]-1,1-dimethyl-pyrrolidiniumbromid (Beispiel 5) wird nach Freisetzen des tertiären Aminoesters die diastereomere Verbindung (3S,2'R)- 3-[(cyclohexylhydroxyphenylacetyl)oxy]-1-methyl-pyrrolidin, D(-)-Hydrogentartrat kristallisiert.

(3S,2'R) 3-[(cyclohexylhydroxyphenylacetyl)oxy]-1-methyl-pyrrolidin, D(-)-Hydrogen-tartrat: ¹H-NMR (300 MHz CD4O): δ (ppm) 1,12-1,2 (M,4H,Cyclohexyl-CH₂), 1,2-1,64 (M,3H,Cyclohexyl-CH₂), 1,64-1,67 (M,2H,Cyclohexyl-CH₂), 1,75-1,85 (M,1H,Cyclohexyl-CH₂), 2,03-2,08 (M,1H,C4), 2,25-2,4 (M,1H,Cyclohexyl-Methin), 2,40-2,55 (M,1H,C4), 2,82 (S,3H,N-Methyl), 3,27-3,34 (M,2H,C5,C2), 3,44 (M,1H,C5), 3,73 (dd,1H,3J=5,45Hz,2J=13,3Hz,C2), 4,42 (S,2H,Tartrat), 5,37 (M,1H,C3), 7,2-7,61 (M,5H) Zuordnung aufgrund H,H-COSY-NMR. ¹³C-NMR (62,5 MHz CD₄O): δ (ppm) (25,31 (t), 26(t), 26,1 (t), 27,32 (t),Cyclohexyl-Methylen), 30,57 (t,C4), 41,1 (q,N-Methyl), 45,6 (d,Cyclohexyl-Methin), 53,93 (t,C5), 59,7 (t,C2), 72,8 (d,Tartrat-Methin), 73,82 (d,C3), 81,3 (s,Hydroxyester C2'), 125,54 (d), 127,2 (d), 127,8 (d), 140,85 (s), 173,64 (s,Tartrat-Carboxyl), 175,8 (s,Hydroxyester-Carboxyl)

Nach Quarternierung mit Methylbromid in tert.Butyl-Methylether erhält man (3S,2'R)-3-[(cyclohexylhydroxyphenylacetyl)oxy]-1,1-dimethyl-pyrrolidiniumbromid. ¹H-NMR (300MHz CD₄O): δ (ppm) (1,1-1,8 (M,10H), Cyclohexyl-Methylen), 2,2-2,4 (M,2H,C4&cHex-Methin), 2,65-2,85 (M,1H,C 4), 3,03 (S,3H,N-Methyl), 3,21 (S,3H,N-Methyl), 3,55-3,8 (M, 3H, C2, C5), 3,86 (dd,1H,C2,²J=13,8Hz,³J=6,1Hz), 5,48 (M,1H,C3), 7,25-7,4 (M,3H), 7,57-7,65 (M,2H). Die Bestimmung der Diastereomerenreinheit (de) erfolgte wie in Beispiel 5 beschrieben.

### Beispiel 7:

### Herstellung von (3R,2'R) 3-[(cyclohexylhydroxyphenylacetyl)oxy]-1,1-dimethyl-pyrrolidiniumbromid 2c (allg.Formel I, Ar = Phenyl, R₁ = Cyclohexyl, R₂ = R₃ = Methyl, n = 1, A = Br)

Herstellung der unter Beispiel 5 aufgeführten Verbindung erfolgt ausgehend von (3R)-1-Methyl-3-pyrrolidinol, 2-Cyclohexyl-2-hydroxy-phenylessigsäuremethylester und NaOMe nach dem unter Beispiel 1 beschriebenen Verfahren. Umesterung und Diastereomerentrennung der 3R-konfigurierten L-(+)-Hydrogentartrate erfolgen in analoger Art und Weise. Zur Quartemierung wird analog der unter Beispiel 5 aufgeführten Vorschrift Methylbromid in tert.Butyl-Methylether verwendet. [¹H]-und [¹³C]-NMR Analytik identisch zu der in Beispiel 5 aufgeführten (3S,2'S)-konngurierten Verbindung. Die Bestimmung der Diastereomerenreinheit (de) erfolgte wie in Beispiel 5 beschrieben.

### Beispiel 8:

### Herstellung von (3R,2'S) 3-[(cyclohexylhydroxyphenylacetyl)oxy]-1,1-dimethyl-pyrrolidiniumbromid 2d (allg.Formel I, Ar = Phenyl, R₁ = Cyclohexyl, R₂ = R₃ = Methyl, n = 1, A = Br)

Aus den Mutterlaugen der Diastereomerentrennung zur Herstellung von (3R,2'R) 3-[(cyclohexylhydroxyphenylacetyl)oxy]-1,1-dimethyl-pyrrolidiniumbromid (Beispiel 7) wird die diastereomere Verbindung (3R,2'S) 3-[(cyclohexylhydroxyphenylacetyl)oxy]-1-methyl-pyrrolidin, L-(+)-Hydrogentartrat kristallisiert. Nach Quarternierung mit Methylbromid in tert.Butyl-Methylether erhält man (3R,2'S) 3-[(cyclohexylhydroxyphenylacetyl)oxy]-1,1-dimethyl-pyrrolidiniumbromid. Die [¹H]- und [¹³C]-NMR analytischen Daten entsprechen jenen der in Beispiel 6 aufgeführten (3S,2'R)-konfigurierten Verbindung. Die Bestimmung der Diastereomerenreinheit (de) erfolgte wie in Beispiel 5 beschrieben.

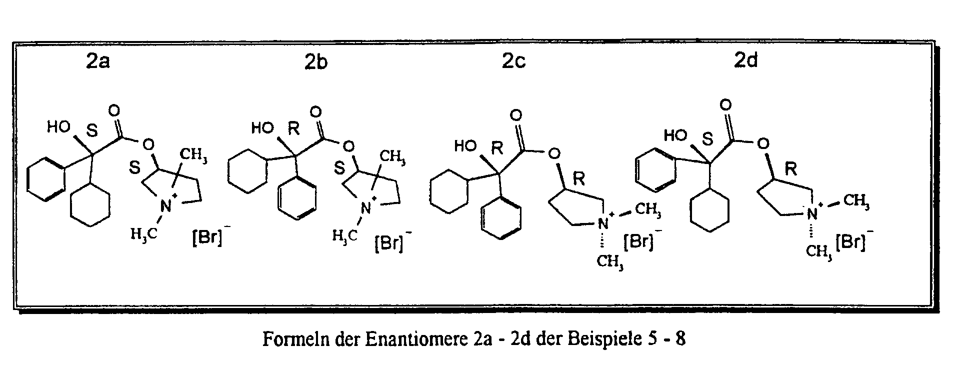

### Beispiel 9:

Pharmakologische Daten von Verbindungen der allg. Strukturformel I mit
AR=Phenyl, R₁ = Cyclopentyl, n= 1, R₂ = R₃ = Methyl, A = Iodid

**Tab.1**

| pharmakologische Affinitätsdaten der Verbindungen Ia-d | | | | | | | |
|---|---|---|---|---|---|---|---|
| Verbindung Abs.Konf.^{§1} | pA₂ | pKᵢ | pA₂^{a} | pKᵢ^{c} | pEC₅₀^{a} | pKᵢ^{b} | pKᵢ^{b} |
| | RVD (M1) | M1 | GPA (M2) | M2 | GPI (M3) | M³ | M4 |
| (3S.2'S) Ia | 8,22 | 8,36 | 7,92 | 7,88 | 6,82 | 7,82 | 7,82 |
| (3S.2'R) Ib | 10,40 | 10,48 | 9,39 | 9,74 | 9,39 | 10,50 | 10,30 |
| (3R.2'R) Ic | 10,30 | 10.18 | 9,43 | 9.63 | 8,76 | 10,2 | 10,27 |
| (3R.2'S) Id | 9,53 | 9,36 | 8,69 | 9,00 | 8,57 | 9,63 | 9,40 |

| | | | | | | | |
|---|---|---|---|---|---|---|---|
| a pA₂ und pEC₅₀ Werte aus funktionellen Experimenten am Kaninchen-Vas-Deferens, Meerschweinchen-Atrium und Meerschweinchen-Ileum. | | | | | | | |
| b pKᵢ-Werte aus [³H]-NMS-Bindungsstudien an M₁-, M-,M₄-Humanrezeptoren aus CHO-K1-Zellen | | | | | | | |
| c pKᵢ-Werte aus [³H]-NMS-Bindungsstudien an M₂-Rezeptoren aus Rattenherzpräparationen d pA₂ = 7,61. | | | | | | | |

Fig. 1 zeigt die pKᵢ-Werte aus [³H]-NMS-Bindungsstudien. Verb. Ia-d gemäß der Tabelle 1.

### Beispiel 10:

Kinetik-Daten am M3-Rezeptorsubtyp von Verbindungen der allg. Strukturformel I mit
AR=Phenyl, R₁ = Cyclopentyl, R₂ = R₃ = Methyl, n= 1, A = Iodid

**Tab.2**

| Assoziations- und Dissoziationskonstanten, Dissoziationshalbwertszeiten am M3-Rezeptorsubtyp | | | |
|---|---|---|---|
| Verb.ME-X¹ | k on^{e} | k off^{f} | t_{½} [min]^{g} |
| (3S,2'S) Ia | 0,052 | 0,8000 | 1 |
| (3S,2'R) Ib | 0,410 | 0,0100 | 70 |
| (3R,2'R) Ic | 0,160 | 0,0060 | 120 |
| (3R,2'S) Id | 0,028 | 0,0080 | 90 |

| | | | |
|---|---|---|---|
| **e** Assoziationskonstante nmol/min, | | | |
| **f** Dissoziationskonstante nmol/min, | | | |
| **g** Dissoziationshalbwertszeit in Minuten. Kinetik-Daten aus NMS-Bindungsstudien an M3-Rezeptorsubtypen aus CHO-K1-Zellinien. **1** Die Zuordnung der Absolutkonfiguration in 2-Position der Hydroxysäuren erfolgte durch Vergleich der CD-Spektren mit den entsprechenden Cyclohexyl-Mandelsäuren. Die Zuordnung der Absolutkonfiguration der 2-Cyclohexyl-2-hydroxy-phenylessigsäuren erfolgte an Hand der Drehwerte gemäß T.D. Inch et.al. J.Chem.Soc 1968 S.1693-1699. | | | |

Fig. 2 zeigt die Dissoziationshalbwertszeiten am M3-Rezeptorsubtyp aus [3H]-NMS-Bindungsstudien. Verb.Ia - Id gemäß der Tabelle.

## Patentansprüche

1. Verwendung eines pharmazeutisch geeigneten Salzes von (3*R*,2'*R*)-3-[(Cyclopentylhydroxyphenylacetyl)oxy]-1,1-dimethyl-pyrrolidinium zur Herstellung eines Arzneimittels zur Behandlung von Spasmen der glatten Muskulatur des Magen-Darm-Kanals sowie zur Behandlung obstruktiver Atemwegserkrankungen.

2. Verwendung eines pharmazeutisch geeigneten Salzes von (3*R*,2'*R*)-3-[(Cyclopentylhydroxyphenylacetyl)oxy]-1,1-dimethyl-pyrrolidinium nach Anspruch 1 zur Herstellung eines Arzneimittels zur Behandlung von Asthma bronchiale

3. Verwendung eines pharmazeutisch geeigneten Salzes von (3*R*,2'*R*)-3-[(Cyclopentylhydroxyphenylacetyl)oxy]-1,1-dimethyl-pyrrolidinium nach Anspruch 1 zur Herstellung eines Arzneimittels zur Behandlung von chronischer Bronchitis

4. Verwendung eines pharmazeutisch geeigneten Salzes von (3*R*,2'*R*)-3-[(Cyclopentylhydroxyphenylacetyl)oxy]-1,1-dimethyl-pyrrolidinium nach Anspruch 1 zur Herstellung eines Arzneimittels mit hoher M₃-Subtypaffinität und langen Dissoziationshalbwertszeiten am M₃-Rezeptorsubtyp zur Behandlung obstruktiver Atemwegserkrankungen.

5. Verwendung eines pharmazeutisch geeigneten Salzes von (3*R*,2'*R*)-3-[(Cyclopentylhydroxyphenylacetyl)oxy]-1,1-dimethyl-pyrrolidinium nach Anspruch 4 zur Herstellung eines Arzneimittels zur Behandlung von Asthma bronchiale

6. Verwendung eines pharmazeutisch geeigneten Salzes von (3*R*,2'*R*)-3-[(Cyclopentylhydroxyphenylacetyl)oxy]-1,1-dimethyl-pyrrolidinium nach Anspruch 4 zur Herstellung eines Arzneimittels zur Behandlung von chronischer Bronchitis

## Claims

1. Use of a pharmaceutically acceptable salt of (3*R*,2'*R*)-3-[(cyclopentylhydroxyphenylacetyl)oxy]-1,1-dimethylpyrrolidinium for preparing a medicament for treating spasms of the smooth musculature of the gastrointestinal tract and for treating obstructive respiratory disorders.

2. Use of a pharmaceutically acceptable salt of (3*R,*2'*R*)-3-[(cyclopentylhydroxyphenylacetyl)oxy]-1,1-dimethylpyrrolidinium according to Claim 1 for preparing a medicament for treating bronchial asthma.

3. Use of a pharmaceutically acceptable salt of (3*R*,2'*R*)-3-[(cyclopentylhydroxyphenylacetyl)oxy]-1,1-dimethylpyrrolidinium according to Claim 1 for preparing a medicament for treating chronic bronchitis.

4. Use of a pharmaceutically acceptable salt of (3*R*,2'*R*)-3-[(cyclopentylhydroxyphenylacetyl)oxy]-1,1-dimethylpyrrolidinium according to Claim 1 for preparing a medicament having high M₃-subtype affinity and long dissociation half-lifes at the M₃ receptor subtype for treating obstructive respiratory disorders.

5. Use of a pharmaceutically acceptable salt of (3*R*,2'*R*)-3-[(cyclopentylhydroxyphenylacetyl)oxy]-1,1-dimethylpyrrolidinium according to Claim 4 for preparing a medicament for treating bronchial asthma.

6. Use of a pharmaceutically acceptable salt of (3*R*,2'*R*)-3-[(cyclopentylhydroxyphenylacetyl)oxy]-1,1-dimethylpyrrolidinium according to Claim 4 for preparing a medicament for treating chronic bronchitis.

## Revendications

1. Utilisation d'un sel pharmaceutiquement acceptable de (3R,2'R)-3-[cyclopentylhydroxyphénylacétyl)oxy]-1,1-diméthylpyrrolidinium pour la préparation d'un médicament pour le traitement de spasmes de la musculature lisse de l'estomac et des voies intestinales ainsi que pour le traitement des maladies obstructives des voies respiratoires.

2. Utilisation d'un sel pharmaceutiquement acceptable de (3*R*,2'*R*)-3-[cyclopentylhydroxyphénylacetyl)oxy]-1,1-dimethylpyrrolidinium selon la revendication 1, pour la préparation d'un médicament pour le traitement de l'asthme bronchial.

3. Utilisation d'un sel pharmaceutiquement acceptable de (3*R*,2'*R*)-3-[cyclopentylhydroxyphénylacétyl)oxy]-1,1-diméthylpyrrolidinium selon la revendication 1, pour la préparation d'un médicament pour le traitement de bronchites chroniques.

4. Utilisation d'un sel pharmaceutiquement acceptable de (3R,2'R)-3-[cyclopentylhydroxyphénylacétyl)oxy]-1,1-diméthylpyrrolidinium selon la revendication 1, pour la préparation d'un médicament avec une importante sélectivité pour les sous types M₃ et une demie durée de dissociation longue sur les sous-types de récepteurs de M₃ pour le traitement de maladies obstructives des voies respiratoires.

5. Utilisation d'un sel pharmaceutiquement acceptable de (3R, 2'R) -3-[cyclopentylhydroxyphénylacétyl)oxy]-1,1-diméthylpyrrolidinium selon la revendication 4, pour la préparation d'un médicament pour le traitement de l'asthme bronchial.

6. Utilisation d'un sel pharmaceutiquement acceptable de (3R,2'R)-3-[cyclopentylhydroxyphénylacétyl)oxy]-1,1-diméthylpyrrolidinium selon la revendication 4, pour la préparation d'un médicament pour le traitement de bronchites chroniques.
